# EUROPEAN PATENT APPLICATION

(11) **EP 1 308 121 A2**
(43) Date of publication of application: **07.05.2003**
(21) Application number: 02256287.0
(22) Date of filing: 11.09.2002
(51) Int. Cl.: A61B 1/005

(54) **Flexible tube and method for manufacturing the same**

(30) Priority: 31.10.2001 JP 2001334784
(71) Applicant: MACHIDA ENDOSCOPE CO., LTD, Bunkyo-ku, Tokyo (JP)
(72) Inventor: Miyagi, Kunihiko, c/o Machida Endoscope Co., Ltd., Tokyo (JP)
(74) Representative: Hallybone, Huw George

(57) **Abstract**

A bent tube 10 includes joint rings 11 spacedly arranged, side by side, in a row and a connection mechanism 20 which is bridgingly disposed between one of the adjacent joint rings 11 and the other. The connecting mechanism 20 has a pair of hinge members 21, 22 which are rotatably connected around a rivet 23 (rotation axis). The hinge 21 is fixed to a groove 11c (hinge attaching portion) formed in one of the adjacent joint rings 11 by soldering, and the hinge member 22 is fixed to a groove lie formed in the other joint ring 11 by soldering. By this, caulking operation, which is necessary for connecting the joint rings, can be performed easily.

## Description

### BACKGROUND OF THE INVENTION

This invention relates to a bent tube used for an endoscope, a catheter and the like, and a method for manufacturing the same.

In general, an endoscope has a main body, an elongated insertion portion extending from the main body, and a bent tube disposed at the tip of the insertion portion. The bent tube includes a plurality of annular joint rings which are arranged, side by side, in a row along the axis. Small protrusion pieces extending towards each other are formed on opposing ends of the adjacent joint rings. Those small protrusions are overlapped in the radial direction of the joint ring and rotatably connected by a rivet (rotation axis). That is, parts (small protrusion pieces) of the adjacent joint rings are mutually overlapped and directly connected together. The rivet is inserted in through holes formed in two small protrusion pieces from the inner side of the joint ring, and the end of the rivet which is projected to the outer periphery is caulked.

In the above conventional direct connection structure, since the rivet is inserted in the joint ring and then inserted in the through holes formed in the small protrusion pieces, the inserting operation is difficult. Moreover, the caulking operation which is performed after the inserting operation is difficult, too. That is, at the time of caulking, it is necessary for the conventional structure that a narrow cantilever rod-like base metal is put into the joint ring and a head portion of the rivet is retained on this base metal. However, it is difficult to withstand the punching force of caulking by such a narrow cantilever rod-like base metal. This often results in insufficient caulking or long time is required for caulking. Moreover, recently, it was demanded to design the endoscope very narrowly and the joint ring was also required to be designed smaller in diameter. For this reason, the base metal is also required to be designed even more narrowly. This means that it becomes more and more difficult to obtain a large strength enough to withstand the caulking.

### SUMMARY OF THE INVENTION

To solve the above problems, the present invention employs a connection mechanism which is separately formed from the joint ring and adjacent joint rings are indirectly connected to each other through this connection mechanism. That is, a bent tube according to the present invention comprises a plurality of joint rings spacedly arranged, side by side, in a row such that every adjacent joint rings have no overlapping, and a connection mechanism bridgingly disposed between one of the adjacent joint rings and the other. A hinge attaching portion is disposed on a peripheral surface of each joint ring. The connection mechanism includes a pair of hinge members rotatably connected to each other around a rotation axis which is disposed orthogonal to the direction where the joint rings are spacedly arranged, side by side. One of the hinge members is fixed to the hinge attaching portion of one of the adjacent joint rings by fixing means such as welding, soldering, bonding or the like, and the other hinge member is fixed to the hinge attaching portion of the other joint ring by the same fixing means. By separately forming the joint ring and the connection mechanism from each other and indirectly connecting the adjacent joint rings through the connection mechanism in the manner as mentioned above, insertion and caulking of the rotation axis can be easily and surely performed. Moreover, one kind of connection mechanism can be used for connecting various kinds of joint rings each having a different diameter, and thus, parts can be commonly used. Moreover, a manufacturing method in which no mold is used can be employed. Owing to this feature, it is not only possible to lower the initial expenses extensively but also to change dimension, etc. easily. Thus, various kinds of bent tubes each having a different diameter and a different degree of bending can be formed easily.

It is preferred that the hinge attaching portion is disposed at an outer peripheral surface of each joint ring. By doing so, the hinge member can easily be attached to the joint ring.

It is also preferred that the hinge attaching portion is a groove formed in a peripheral surface of each joint ring and that the hinge member is fitted into the groove. By making the hinge attaching portion in the form of a groove, the hinge can easily be positioned and the attaching precision can be enhanced. Moreover, by dropping the hinge member into the groove, the hinge member can be prevented from projecting in the radial direction from the joint ring and thus, it can be prevented from being caught by other component elements.

It is preferred that a number, four, of the hinge attaching portions are arranged at an interval of 90 degrees in a circumferential direction of the joint ring and a pair of the connecting mechanisms are arranged 180 degrees away from each other in the circumferential direction and displaced by 90 degrees from the adjacent connecting mechanisms in the circumferential direction with the joint ring disposed therebetween. Owing to this feature, a bent tube bendable in four directions can be constituted.

It is also preferred that a wire guide portion for guiding a control wire for bending the bent tube is integrally formed on an inner periphery of the joint ring, the joint ring is formed of a softer material than the control wire and the hinge members are each formed of a material having a larger strength than the joint ring, thereby reinforcing the joint ring. By integrally forming the wire guide portion on the joint ring, it becomes unnecessary that the wire guide is separately formed and then attached. By forming the joint ring including the wire guide from a softer material than the control wire, the control wire can be prevented from getting damaged and thus, it can be prevented that the bending control becomes impossible to make due to cut-off of the wire. Moreover, by forming the hinge member from a material having a larger strength than the joint ring, the joint ring formed from the soft material can be reinforced.

In a method according to the present invention, at the time for manufacturing a bendable bent tube as a whole by arranging a plurality of joint rings, side by side, in a row, a first and a second hinge member are formed separately from the joint ring. The first and second hinge members are rotatably connected to each other around a rotation axis which is disposed orthogonal to the direction where the joint rings are spacedly arranged, side by side. Thereafter, one of adjacent joint ring of the plurality of joint rings is fixed to the first hinge member by fixing means such as welding, soldering, bonding or the like and the other joint ring is fixed to the second hinge member by the same fixing means. Owing to this arrangement, the rotation axis can easily and surely be inserted and caulked. Moreover, parts can be commonly used. In addition, a manufacturing method in which no mold is used can be employed. Owing to this feature, it is not only possible to lower the initial expenses extensively but also to change dimension, etc. easily. Thus, various kinds of bent tubes each having a different diameter and a different degree of bending can be formed easily.

It is preferred that the first and second hinge members are formed by etching. By doing so, the hinge member can be manufactured at a low cost and in large number at a time. Moreover, dimension can be changed easily and inexpensively.

### BRIEF DESCRIPTION OF THE DRAWINGS

FIG. 1 is a side view of a bent tube of an endoscope according to one embodiment of the present invention.
FIG. 2 is a sectional view of the bent tube taken on line II-II of FIG. 1.
FIG. 3 is a front view of a joint ring of the bent tube.
FIG. 4 is a side view of the joint ring taken on line IV-IV of FIG. 3.
FIG. 5 is a sectional view of the bent tube taken on line of V-V of FIG. 2.
FIG. 6 is a plan view of a connection mechanism of the bent tube.
FIG. 7 is a sectional view of the connection mechanism taken on line VII-VII of FIG. 6.
FIG. 8 is a plan view showing a first and a second hinge member of the connection mechanism, which is in a midway of the manufacturing process, after an etching step but before a bending step.
FIG. 9 is an explanatory view showing the manner for caulking a rivet inserted into the first and the second hinge member.
FIG. 10 is an explanatory view showing the manner for connecting together adjacent joint ring by the connection mechanism.
FIG. 11 is a schematic view of the endoscope.

### DETAILED DESCRIPTION OF THE INVENTION

One embodiment of the present invention will now be described with reference to the accompanying drawings.

FIG. 11 shows an endoscope S. The endoscope S includes a main body 1 and a flexible insertion portion 2 extending from this main body 1. The main body 1 is provided with an eyepiece portion 3 and a bending control handle 4. The insertion portion 2 is provided at a tip thereof with a bent portion 5 which is remote controlled by the handle 4. The bent portion 5 is provided at a tip thereof with a tip component portion 6. An image light made incident through an observation window (not shown) formed in this tip component portion 6 is transmitted to the eyepiece portion 3 through which the image can be observed.

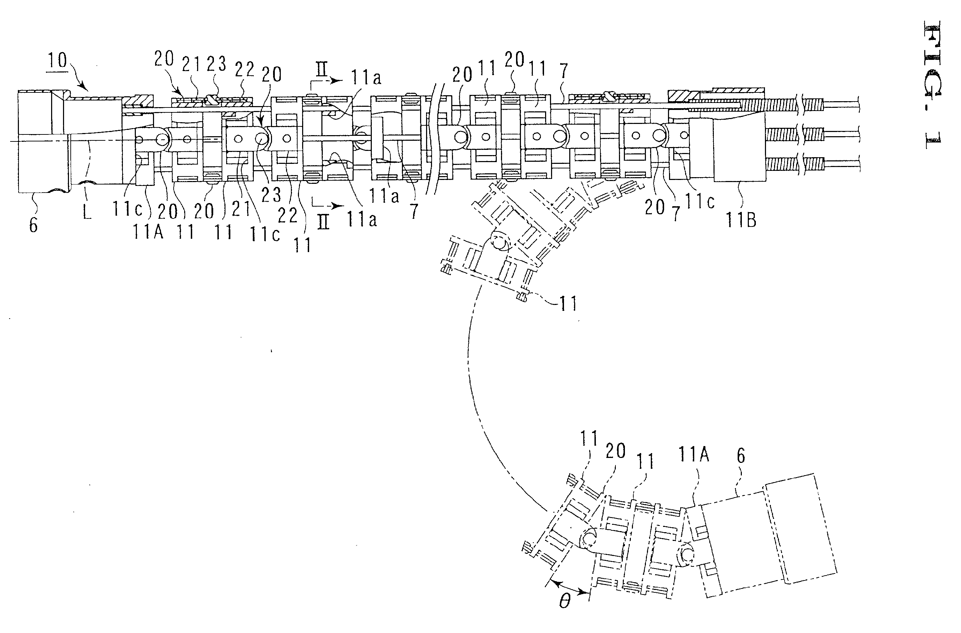

A bent tube 10 according to the present invention is received in the bent portion 5 of the endoscope S. As shown in FIG. 1, the bent tube 10 includes a number of (plurality of) joint rings 11 which are arranged, side by side, in a row along an axis L. Every adjacent joint rings 11 are indirectly connected to each other through a connection mechanism 20 which is formed separately from the joint rings 11.

Specifically, all the joint rings 11 are designed to have a same configuration and a same dimension only excepting two, one being located nearest to the basal end side and connected to the insertion portion 2, and the other being located nearest to the distal end side and integral with the tip component portion 6. The joint rings located nearest to the distal end side and the basal end side are hereinafter denoted by 11A, and 11B, respectively, where necessary.

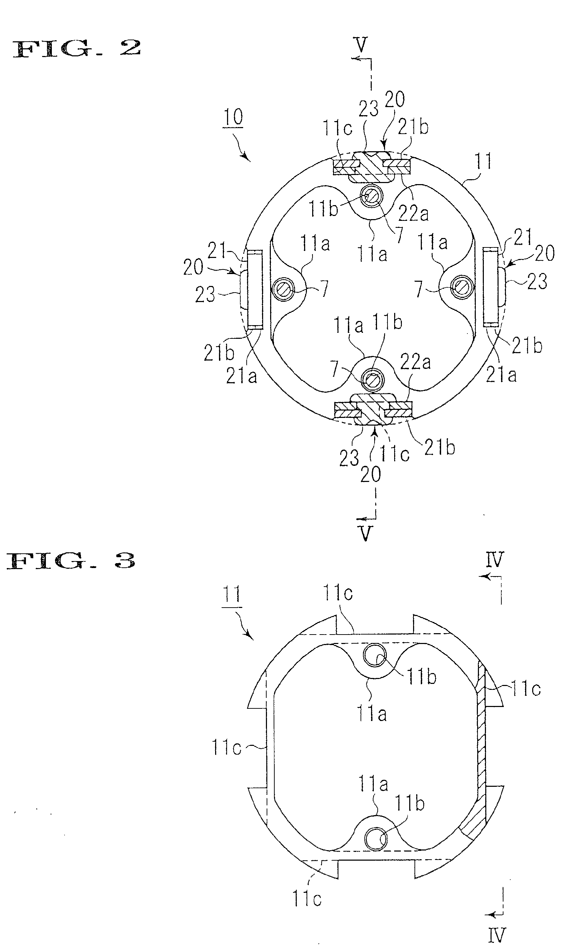

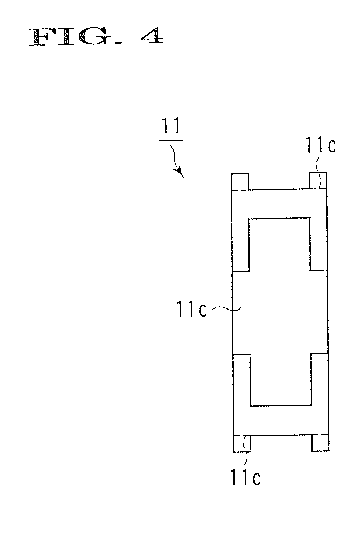

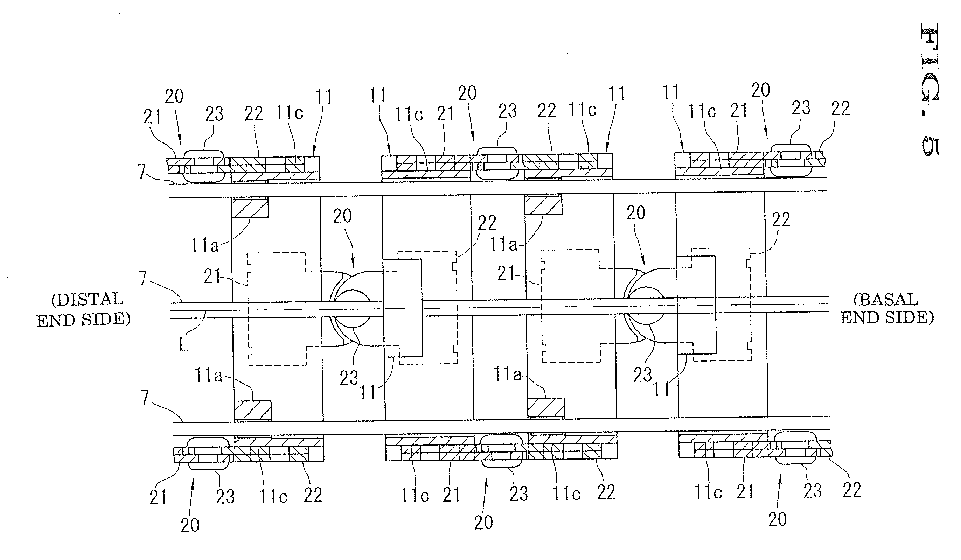

As shown in FIGS. 3 through 5, each joint ring 11 is formed in a short cylindrical configuration (short tubular configuration). As a material of the joint ring 11, brass is used.

Generally round wire guide portions 11a are integrally formed on an inner periphery of each joint ring 11 in such a manner as to project radially inward. A guide hole 11b is formed in each wire guide portion 11a in such a manner as to extend therethrough in a direction of the axis L. A periphery side surface of the wire guide portion 11a is rounded and smoothly continuous with an inner peripheral surface of the joint ring 11. A distal end face of the wire guide portion 11a is flush with a distal end face of the joint ring 11 and a base end face thereof is located in an intermediate position in the axial direction of the joint ring 11.

One pair of the wire guides 11a are arranged 180 degrees away from each other in a circumferential direction of the joint ring 11. As shown in FIGS. 1, 2 and 5, the corresponding wire guide portions 11a in every adjacent joint rings 11 are displaced by 90 degrees in the circumferential direction. Owing to this arrangement, the corresponding wire guide portions 11a of every other joint rings 11 are arranged in a row along the axis L. A control wire 7 is inserted in the guide holes 11b of those wire guide portions 11a which are arranged in a row. A basal end of this control wire 7 is connected to the control handle 4 of the endoscope main body 1 and a distal end thereof is fixed to the joint ring 11A. In the bent tube 10, a number, four, of the rows of the wire guide portions 11a arranged in the direction of the axis L are provided, and a number, four, of the control wires 7 are provided in correspondence the number of the rows of the wire guide portions 11a.

Since a large tensile strength is required, the control wire 7 is composed of a stranded wire of stainless steel. On the other hand, the joint ring 11 and thus, the wire guide portion 11a are composed of a brass which is a comparatively soft material as previously mentioned. Accordingly, when friction occurs between the control wire 7 and the wire guide portion 11a, the wire guide portion 11a is worn out. By this, the control wire 7 can be prevented from getting damaged and thus, it can be prevented that the bending control becomes impossible to make due to cut-off of the wire 7.

As shown in FIGS. 3 through 5, grooves 11c (hinge attaching portions) are formed in an outer periphery of the joint ring 11. Those grooves 11c are formed by cutting flatly the outer peripheral surface of each joint ring 11 into a cruciform along the direction of the axis L and the circumferential direction. A bottom surface of the groove 11c is flat. The flattened portion in the direction of the axis L in the groove 11c is continuous with opposite end faces of the joint ring 11. A number, four, of the grooves 11c are arranged at an interval of 90 degrees in the circumferential direction of the joint ring 11. Of those four grooves 11c, two grooves 11c which are away from each other by 180 degrees in opposing relation are arranged in the same circumferential positions as the pair of wire guide portions 11a, while the remaining two grooves 11c are arranged in such a manner as to be displaced by 90 degrees with respect to the wire guide portions 11a. In the joint rings 11A and 11B, only two grooves 11c which are away from each other by 180 degrees in opposing relation are formed.

The groove 11c is adapted to fix the connection mechanism 20 thereto.

The connection mechanism 20 will now be described in detail.

As shown in FIGS. 2, 6 and 7, the connection mechanism 20 includes a pair of first and second hinge members 21, 22 which is separately formed from the joint ring 11, and a rivet 23 (rotation axis) for connecting the hinge members 21, 22 together. Along the axis L of the bent tube 10, the first hinge member 21 is disposed at the distal end side, and the second hinge member 22 is disposed at the basal end side (see FIG. 1).

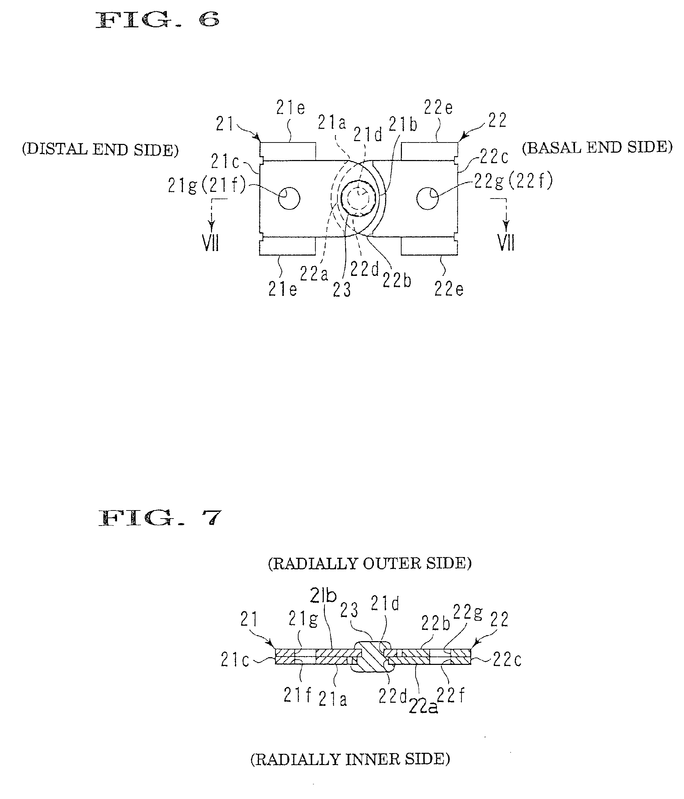

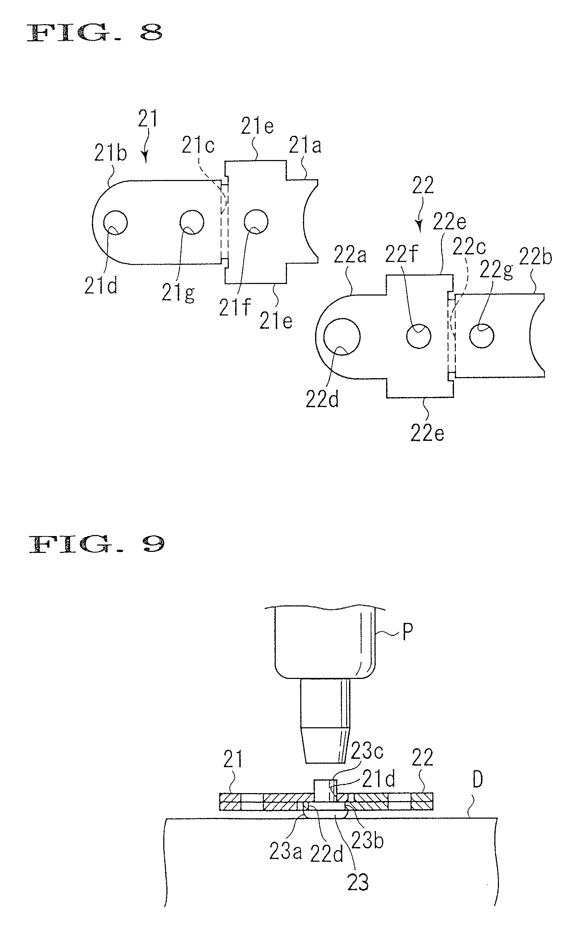

The first hinge member 21 includes an inner plate piece 21a and an outer plate piece 21b overlapped on the outside of the inner plate piece 21a in the radial direction. As shown in FIG. 8, this first hinge member 21 is formed by overlappingly folding a thin stainless steel plate piece, which is flat as a whole, into two and then welding the same. A bent portion 21c formed at that time serves as a border between the plate pieces 21a, 21b. Positioning holes 21f, 21g for the use at the time of folding are formed respectively in the plate 21a, 21b, at positions which are in line symmetrical relation with respect to the bent portion 21c. As shown in FIGS. 6 and 7, in a folded complete state, the outer plate piece 21b is more projected towards the basal end side than the inner plate piece 21a. A rivet insertion hole 21d is formed in this projected portion of the outer plate piece 21b.

Likewise, the second hinge member 22 is formed by overlappingly folding a thin stainless steel plate piece and then welding the same. The second hinge member 22 is divided into an inner plate piece 22a and an outer plate piece 22b serving the bent portion 22c as a border. Positioning holes 22f, 22g are formed in those plate pieces 22a, 22b, respectively. In a folded complete state, the inner plate piece 22a is more projected towards the distal end side than the outer plate piece 22b. A rivet insertion hole 22d is formed in this projected portion of the inner plate piece 22a. The rivet insertion hole 22d of the second hinge member 22 is larger in diameter than the rivet insertion hole 21d of the first hinge member 21.

As best shown in FIG. 7, in the connection mechanism 20, the inner plate pieces 21a, 22a of the first and second hinge members 21, 22 are arranged on a same plane, while the outer plate pieces 21b, 22b are arranged on a same plane. The projected portion of the outer plate piece 21b of the first hinge member 21 and the projected portion of the inner plate piece 22a of the second hinge member 22 are overlapped. In that condition, the rivet insertion holes 21d, 22d formed in those projected portions are aligned. The rivet 23 is inserted into the aligned holes 21d, 22d from the inner side in the radial direction.

As shown in FIG. 9, a leg portion of the rivet 23 includes an enlarged diameter portion 23b on the side of a head portion 23a and a reduced diameter portion 23c continuous with a forward end thereof. Thus, the leg portion of the rivet 23 is stepped. The enlarged diameter portion 23b is rotatably inserted in the insertion hole 22d, and the reduced diameter portion 23c is inserted in the insertion hole 21d. As shown in FIG. 7, a projected end of this reduced diameter portion 23c from the insertion hole 21d is caulked to the plate piece 21b. By this, two hinge members 21, 22 are rotatably connected together about the rivet 23. By sandwiching the plate piece 21b between the caulked portion and the step formed between the enlarged diameter portion 23b and the reduced diameter portion 23c, the rivet 23 is prohibited from playing in the radial direction of the bent tube 10.

As shown in FIGS. 1, 5 and 10, the connection mechanism 20 is bridgingly disposed between the adjacent two joint rings 11. A basal end side portion of the second hinge member 22 is fitted in the groove 11c formed in the joint ring 11 on the basal end side. In that condition, a pair of collars 22e provided on the inner plate piece 22a is abutted with an edge of the groove 11c along the circumferential direction. By this, the second hinge member 22 and the basal end side joint ring 11 are correctly positioned in the direction of the axis L. The plate pieces 22a, 22b, which are more on the distal end side than the collars 22e, are abutted with the edge of the groove 11c in the direction of the axis L. By this, the second hinge member 22 and the basal end side joint ring 11 are correctly positioned in the circumferential direction. The second hinge member 22 and the joint ring 11 are fixed together by soldering (fixing means).

Likewise, the distal end side portion of the first hinge member 21 is fitted in the groove 11c formed in the joint ring 11 on the distal end side. In that condition, a pair of collars 21e provided on the inner plate piece 21a is abutted with an edge of the groove 11c along the circumferential direction and the plate pieces 21a, 21b, which are more on the basal end side than the collars 21e, are abutted with the edge of the groove 11c along the direction of the axis L. By this, the first hinge member 21 and the distal end side joint ring 11 are correctly positioned in the direction of the axis L and in the circumferential direction. The first hinge member 21 and the joint ring 11 are fixed together by soldering (fixing means).

Owing to the above-mentioned arrangement, the adjacent two joint rings 11 are indirectly connected to each other through the connection mechanism 20. In that condition, the adjacent two joint rings 11 do not have any mutually overlapped portion and are spacedly arranged side by side.

As shown in FIGS. 1, 2 and 5, a number, two, of the connection mechanisms 20 are arranged 180 degrees away from each other between the adjacent joint rings 11. Moreover, each connection mechanism 20 is displaced by 90 degrees in the circumferential direction from the corresponding connection mechanism 20 which is adjacent thereto in the direction of the axis L with only one joint ring 11 sandwiched therebetween. In one joint ring 11, the two first hinge members 21 of the connection mechanism 20 for the joint ring 11, which is more on the basal end side than the above-mentioned one joint ring 11, are fitted in the two grooves 11c which are 180 degrees away from each other, and the two second hinge members 22 of the connection mechanism 20 for the joint ring 11, which is more on the distal end side than the above-mentioned one joint ring 11, are fitted in the remaining two grooves 11c.

Owing to the above arrangement, the bent tube 10, as a whole, can be bent in four directions. That is, when the control handle 4 of the endoscope main body 1 is turned, selected one of the four control wires 7 is pulled towards the basal end side and the bent tube 10 is bent towards the side of such selected wire 7 (see the imaginary line of FIG. 1).

A method for manufacturing the bent tube 10 thus constructed will now be described.

First, the joint ring 11 is made by machining a brass. Since the joint ring 11 does not have the thin and flat small protrusion piece which the conventional direct connection mechanism has, a machining tool of the type which requires no mold can be used for machining the same, such as an engraving machine, a milling machine such as a cutting machine tool, a laser beam machine, or the like. Accordingly, the initial expenses can be lowered extensively compared with the conventional case where the joint ring is formed by a mold. Moreover, the dimension such as the outer diameter can easily be made, and thus, bent tubes having various kinds of sizes can easily and inexpensively be made. In addition, since brass is used as its material, a good machining performance can be obtained.

Furthermore, since the wire guide portion 11a is integrally provided on the joint ring 11, it is no more required that the wire guide is separately made and then attached to the joint ring 11.

The connection mechanism 20 is made separately from the joint ring 11. That is, first, a thin and flat plate of stainless steel which is to be formed into the first and second hinge members 21, 22 is prepared. A resist photoengraving modeling the basic patterns of the hinge members 21, 22 is formed on this flat plate and then subjected to etching with a strong acid. By doing so, a plurality of the hinge members 21, 22 can be obtained, which are before subjected to the bending step, as shown in FIG. 8. The resist photoengraving is very inexpensive compared with the press mold, and the initial expenses can be lowered extensively as in the case with the joint ring 11. Moreover, since the dimension such as length, of the hinge members 21, 22 can easily be changed, the distance between the joint rings 11 can freely be adjusted, and thus, an angle θ (see FIG. 1), i.e., degree of bending of the bent tube 10 can freely be changed.

At the time of etching, a half-etching is preliminarily applied to the area serving as the folding portions 21c, 22c of the hinge members 21, 22. By doing so, the thickness of the area is reduced and the hinge members 21, 22 can easily be folded. The plate pieces 21a, 21b or the plate pieces 22a, 22b which are overlapped by bending, are spot welded. By this, the hinge members 21, 22 are finished.

Then, as shown in FIG. 9, the rivet insertion holes 21d, 22d of the hinge members 21, 22 are aligned and the rivet 23 is inserted therein. At that time, since the hinge members 21, 22 are of a flat plate-like configuration and not yet attached with the joint rings 11, the inserting operation of the rivet 23 can very easily be performed.

In that inserted condition, the hinge members 21, 22 are placed on a base metal D and the head portion 23a of the rivet 23 is abutted with the base metal D. Since the hinge members 21, 22 are not yet attached with the joint rings 11, the base metal D can be designed large irrespective of the dimension of the joint rings 11 and thus, the base metal D can have a sufficiently large strength. By this, the leg portion 23c of the rivet 23 can easily and surely be caulked by a punch P, and thus, the caulking precision can be enhanced. In this way, the connection mechanism 20 is finished.

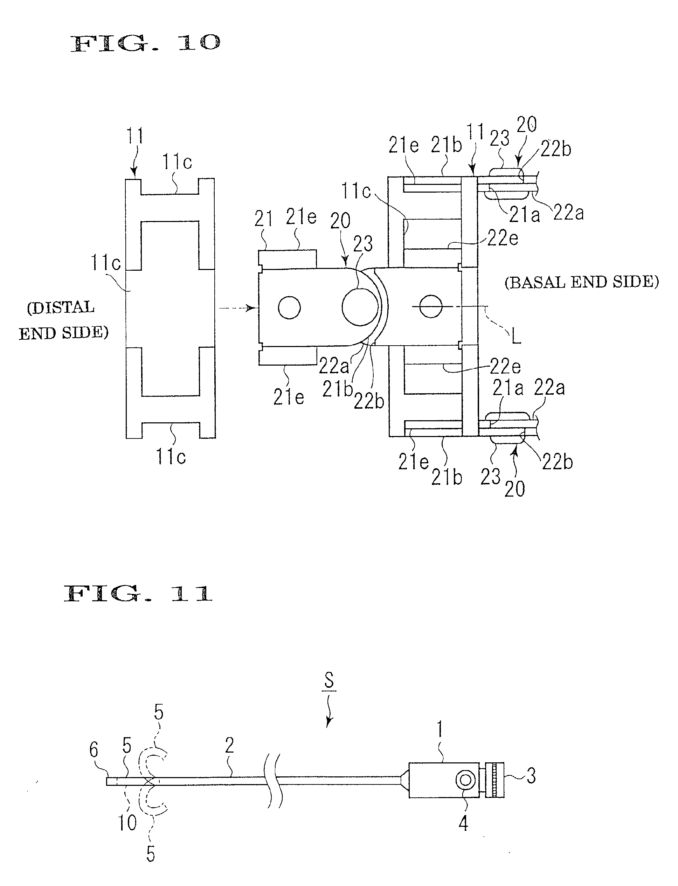

The adjacent joint rings 11 are connected to each other using the finished connection mechanism 20. That is, as shown in FIG. 10, the second hinge member 22 of the connection mechanism 20 is fitted in the groove 11c formed in the basal end side joint ring 11. At that time, the positioning of the hinge member 22 in the circumferential direction and in the direction of the axis L can be performed through the groove 11c and thus, the attaching precision can be enhanced. Moreover, the fixing strength of the second hinge member 22 can be enhanced. Then, the hinge member 22 is fixed to the joint ring 11 by soldering. Likewise, the first hinge member 21 is fitted in the groove 11c formed in the distal end side joint ring 11 and soldered. The attaching precision and the fixing strength of the first hinge member 21 can be enhanced by the groove 11c as in the case described above.

As seen, by dropping the hinge members 21, 22 into the grooves 11c, the entire connection mechanism 20 including the rivet 23 can be prevented from projecting from the outer periphery (imaginary line of FIG. 2) of the joint ring 11 where no groove 11c is presumed to be formed as shown in FIG. 2. Owing to this arrangement, the connection mechanism 20 can be prevented from being caught by an externally attached tube, etc. of the bent tube 10.

Moreover, by providing the stainless steel-made hinge members 21, 22 having a high rigidity are provided to the peripheral surface of the joint ring 11 formed of a soft brass, the strength of the joint ring 11 can be reinforced. Especially, in the bent tube 10 which can be bent in four directions, since the hinge members 21, 22 are provided to four positions of the joint ring 11, the reinforcing degree of the joint ring 11 can even more be enhanced.

In this way, by sequentially jointing the joint rings 11 through the connection mechanisms 20, the bent tube 10 can be finished.

Since the connection mechanism 20 is separately formed from the joint ring 11, for example, one kind of connection mechanisms can be used for connecting various kinds of joint rings each having a different diameter, and thus, parts can be commonly used. Moreover, the same kind of joint rings can be combined with connection mechanisms each having a different length, and thus, various kinds of bent tubes can easily be made.

The present invention is not limited to the above embodiment but many changes and modifications can be made. For example, the present invention can be applied not only to the endoscope but also to a catheter and other devices. It can be applied not bent tubes which can be bent not only in four directions but also in two or one direction. The hinge attaching portion may be formed on the inner peripheral surface of the joint ring. Instead of soldering, the hinge member and the joint ring may be fixed together by other fixing means such as welding, for example, spot welding, bonding using an adhesive agent, etc. The material of the joint ring is not limited to metal such as brass. Plastics, ceramics, etc. may be used as its material. Plastics and ceramics are rich in machinability and anti-corrosion. In addition, they are light in weight. Accordingly, the bent tube can be made light in weight. The material of the hinge member is not limited to stainless steel, either.

## Claims

1. A bent tube(10) comprising a plurality of joint rings(11) arranged, side by side, in a row,
**CHARACTERIZED in that** said plurality of joint rings(11) being spacedly arranged such that adjacent joint rings(11) have no overlapping, and a connection mechanism(20) being bridgingly disposed between one of said adjacent joint rings(11) and the other, a hinge attaching portion(11c) being disposed on a peripheral surface of each joint ring(11), said connection mechanism(20) including a pair of hinge members(21,22) rotatably connected to each other around a rotation axis(23) which is disposed orthogonal to the direction where said joint rings(11) are spacedly arranged, side by side, one(21) of said hinge members(21,22) being fixed to the hinge attaching portion(11c) of one of said adjacent joint rings(11) by fixing means such as welding, soldering, bonding or the like, and the other hinge member(22) being fixed to the hinge attaching portion(11c) of the other joint ring(11) by the same fixing means.

2. A bent tube(10) according to claim 1, wherein the hinge attaching portion(11c) is disposed at an outer peripheral surface of each joint ring(11).

3. A bent tube(10) according to claim 1, wherein the hinge attaching portion(11c) is a groove(11c) formed in a peripheral surface of each joint ring(11) and said hinge member(21/22) is fitted into said groove(11c).

4. A bent tube(10) according to claim 1, wherein a number, four, of the hinge attaching portions(11c) are arranged at an interval of 90 degrees in a circumferential direction of said joint ring(11) and a pair of said connecting mechanisms(20) are arranged 180 degrees away from each other in the circumferential direction and displaced by 90 degrees from the adjacent connecting mechanisms(20) in the circumferential direction with said joint ring(11) disposed therebetween.

5. A bent tube(10) according to claim 1, wherein a wire guide portion(11a) for guiding a control wire(7) for bending said bent tube(10) is integrally formed on an inner periphery of said joint ring(11), said joint ring(11) is formed of a softer material than said control wire(7) and said hinge members(20) are each formed of a material having a larger strength than said joint ring(11), thereby reinforcing said joint ring(11).

6. A method for manufacturing a bent tube(10) comprising at the time for manufacturing a bendable bent tube(10) as a whole by arranging a plurality of joint rings(11), side by side, in a row, forming a first and a second hinge member(21,22) separately from said joint ring(11), rotatably connecting said first and second hinge members(21,22) around a rotation axis(23) which is disposed orthogonal to the direction where said joint rings(11) are spacedly arranged, side by side, and thereafter, fixing one of adjacent joint ring(11) of said plurality of joint rings(11) to said first hinge member(21) by fixing means such as welding, soldering, bonding or the like and fixing the other joint ring(11) to said second hinge member(22) by the same fixing means.

7. A method for manufacturing a bent tube(10) according to claim 6, wherein said first and second hinge members(21,22) are formed by etching.
